# EUROPÄISCHE PATENTSCHRIFT

(11) **EP 0 641 382 B1**
(45) Veröffentlichungstag und Bekanntmachung des Hinweises auf die Patenterteilung: **03.01.1996**
(21) Anmeldenummer: 93909915.6
(22) Anmeldetag: 07.05.1993
(51) Int. Cl.: C12N 1/00

(54) **VERFAHREN ZUR KONTINUIERLICHEN ZUBEREITUNG EINES STERILEN NÄHRMEDIUMS**
CONTINUOUS PREPARATION PROCESS OF A STERILE CULTURE MEDIUM
PROCEDE DE PREPARATION CONTINUE D'UN MILIEU NUTRITIF STERILE

(30) Priorität: 09.05.1992 DE 4215339
(43) Veröffentlichungstag der Anmeldung: 08.03.1995
(73) Patentinhaber: Westfalia Separator AG, D-59302 Oelde (DE)
(72) Erfinder: MANNWEILER, Klaus, D-4740 Oelde (DE); KUCHENBECKER, Ralf, D-5810 Witten (DE); ROSENTHAL, Werner, D-5870 Hemer (DE)
(86) Internationale Anmeldenummer: EP9301118
(87) Internationale Veröffentlichungsnummer: WO9323525

(56) Entgegenhaltungen:
- BE-A- 468 817
- GB-A- 719 886
- BIOTECHNOLOGY AND BIOENGINEERING. Bd. 18, 1976, NEW YORK US Seiten 1433 - 1443 V.F. LARSEN ET AL. 'Automatic membrane-filtration system for the "on-demand" supply of large volumes of sterile medium in continuous culture.'
- BIOSIS abstract no. 94109248,O. Rodas-Suarez et al."Substance produced by Klebsellia-pneumoniae K8 which protects it from the bactericidal effect of human normal serum. & Revista Latinoamericana de Microbiologia, 33 (1), 1991, s. 1-5.

## Beschreibung

Die Erfindung bezieht sich auf ein Verfahren zur kontinuierlichen Zubereitung eines sterilen Nährmediums, bei dem verschiedene Medienkomponenten zu einem Medienstrom vermischt werden, der zur Beschickung eines Bioreaktors geeignet ist.

Die verfahrenstechnischen Stufen eines kontinuierlich betriebenen Bioreaktors bestehen im wesentlichen aus Nährmediensterilisation, Fermentation und Produktaufbereitung bzw. Produktanalytik. Der erfolgreiche kontinuierliche Betrieb eines Bioreaktors ist unter anderem von der Qualität des steril zulaufenden Nährmediums abhängig. Für dessen Herstellung sind drei Verfahren bekannt, und zwar das High Temperature Short Residence Time (HTSRT-) Verfahren, die Sterilfiltration und die Benutzung kontaminationsunempfindlicher Medien.

Beim HTSRT-Verfahren wird der insterile Nährmedienstrom in einem Wärmeaustauschersystem auf ca. 135 °C erhitzt und für wenige Minuten bei dieser Temperatur gehalten. Dies führt zur Abtötung der vorhandenen Mikroorganismen, und man erhält so ein steriles Nährmedium. Obwohl sehr effektiv bezüglich der Keimabtötung, hat das Verfahren den Nachteil, daß einerseits verschiedene Medienbestandteile miteinander reagieren können, z. B. Zuckermoleküle mit Aminosäuren zu Melanoiden, und daß andererseits bestimmte Medienbestandteile, beispielsweise Vitamine, bei den hohen Temperaturen zerstört werden können.

Bei der Sterilfiltration wird durch den Einbau eines Sterilfilters in die Zulaufleitung zum Bioreaktor ein steriles Nährmedium erzeugt. Da bei diesem Verfahren nicht mit hohen Temperaturen gearbeitet werden muß, ergibt sich eine hohe Qualität des Nährmediums. Bei der kontinuierlichen Verarbeitung vieler gängiger Nährmedien können die Filter jedoch schon nach relativ kurzer Zeit zuwachsen. Ein sicherer und kontaminationsfreier Betrieb ist damit nicht gewährleistet, so daß dieses Verfahren in der Praxis vergleichsweise wenig angewandt wird.

BIOTECHNOLOGY AND BIOENGINEERING (1976) 18: 1433 beschreibt eine automatische Membranfiltration, bei der die Blockierung des Sterilfilters durch das Vorschalten eines nicht-sterilen, leicht autauschbaren Vorfilters vermieden wird.

Bei der Benutzung kontaminationsunempfindlicher Medien ergibt sich automatisch ein steriles Nährmedium, da aufgrund der Zusammensetzung ein Wachstum von Kontaminanten nicht möglich ist. Als Anwendungsgebiete eignen sich aber nur Fermentationsverfahren, die im stark sauren Milieu ablaufen oder die Antibiotika im Nährmedium enthalten. Der Einsatz solcher Verfahren ist jedoch begrenzt.

Der Erfindung liegt die Aufgabe zugrunde, ein Verfahren zur kontinuierlichen Zubereitung eines sterilen Nährmediums zu schaffen, das universell einsetzbar ist, eine fortdauernde kontinuierliche Fahrweise ermöglicht und eine schonende Behandlung der Nährmedien garantiert.

Diese Aufgabe wird dadurch gelöst, daß die Medienkomponenten vor der Sterilisation miteinander vermischt werden, der so erzeugte Medienstrom einem Querstromfiltrationsmodul zugeführt wird, das mit einer Filtrationsmembran versehen ist, durch die der Medienstrom aufgeteilt wird in Permeat und Retentat, das Retentat einer Zentrifuge zwecks Abscheidung von Keimen zugeführt wird und das zentrifugierte Retentat zurückgegeben wird in den Medienstrom zum Querstromfiltrationsmodul, während das Permeat dem Bioreaktor eingespeist wird.

Bei dem Verfahren können die Medienkomponenten schon im nichtsterilen Bereich miteinander vermischt werden, wodurch sich ein einfaches Handhaben der Anlage und eine hohe Flexibilität ergibt, was die Kombinationsmöglichkeiten der verschiedenen Medienkomponenten betrifft. Durch die Zentrifuge werden ständig Mikroorganismen aus dem Retentat-Kreislauf ausgeschleust, wodurch ein Zu- bzw. Durchwachsen der Filtrationsmembran vermieden wird. Außerdem sorgt die Zentrifuge selbsttätig für die Aufrechterhaltung einer ausreichenden Rezirkulationsströmung, durch die eine unvorteilhafte Ablagerung von Mikroorganismen auf der Filtrationsmembran unterbleibt. Des weiteren wird durch die Zentrifuge ein ausreichender Transmembrandruck erzeugt.

Bei einer vorteilhaften Ausgestaltung des Verfahrens wird die Temperatur des Medienstromes nach der Zugabe des Retentates durch einen Wärmetauscher auf eine Temperatur von mindestens 70 °C eingestellt. Hierdurch werden einerseits viele Keime abgetötet und andererseits das Zellwachstum thermoresistenter Keime so stark vermindert, daß es nicht zu einer Überlastung von Zentrifuge und Querstromfiltrationsmodul kommen kann. Durch die moderate Temperatur wird eine Schädigung von Medienbestandteilen vermieden.

Gemäß einer weiteren vorteilhaften Ausgestaltung des Verfahrens beträgt der Porendurchmesser der Filtrationsmembran maximal 0,2 Mikrometer. Es hat sich gezeigt, daß bei diesem Durchmesser einerseits hohe Volumenströme des Permeats erreicht werden und andererseits die Sterileigenschaften des Moduls ausreichend sind. Die besten Ergebnisse wurden mit einer Filtrationsmembran erzielt, deren Porendurchmesser 0,14 Mikrometer betrug.

Zur Erzielung einer ausreichend langen Standzeit des Querstromfiltrationsmoduls sollte der Volumenstrom des Retentates mindestens genau so groß sein wie der Volumenstrom des Permeates. Vorzugsweise sollte der Volumenstrom des Retentates mindestens viermal so groß sein wie der Volumenstrom des Permeates.

Bei Verwendung von Verdrängerpumpen zur Dosierung der Medienkomponenten läßt sich das Verfahren besonders einfach regelungstechnisch durchführen.

Bei einer weiteren vorteilhaften Ausgestaltung des Verfahrens wird der Ablaufdruck der Zentrifuge konstant gehalten durch die Regelung des Volumenstromes einer Medienkomponente.

Die Volumenströme der übrigen Medienkomponenten werden vorteilhaft dem geänderten Volumenstrom der geregelten Medienkomponente angepaßt.

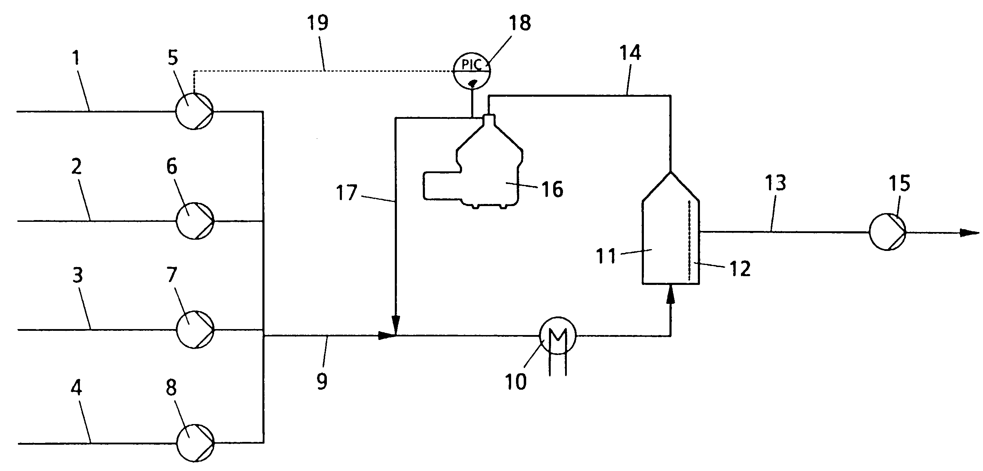

Ein Ausführungsbeispiel der Erfindung ist in der Zeichnung dargestellt und wird nachstehend näher erläutert.

Mit 1, 2, 3 und 4 sind in der Zeichnung die unterschiedlichen Medienkomponenten bezeichnet, die mittels Verdrängerpumpen 5, 6, 7 und 8 zu einem Medienstrom 9 vermischt werden, der in einem Wärmetauscher 10 aufgeheizt wird, bevor er ein Querstromfiltrationsmodul 11 passiert. Im Querstromfiltrationsmodul 11 befindet sich eine Filtrationsmembrane 12, die den Medienstrom 9 aufteilt in Permeat 13 und Retentat 14. Das sterile Permeat 13 wird mittels Pumpe 15 einem nicht dargestellten Bioreaktor zugeführt. Das Retentat 14 wird in eine Zentrifuge 16 geleitet, in der ständig Mikroorganismen aus dem Retentat 14 abgeschieden werden. Das aus der Zentrifuge 16 ablaufende gereinigte Retentat 17 wird wieder mit dem Medienstrom 9 vor Eintritt in den Wärmetauscher 10 vermischt. In der Leitung für das gereinigte Retentat 17 ist ein Druckregler 18 vorgesehen, der über eine Leitung 19 mit der Pumpe 5 in Verbindung steht.

Die Medienkomponenten 1, 2, 3 und 4 werden mittels der Pumpen 5, 6, 7 und 8 im nichtsterilen Bereich miteinander im gewünschten Verhältnis zum Medienstrom 9 vereinigt, Dadurch ergibt sich ein einfaches Handhaben der Anlage und eine hohe Flexibilität, was die Kombinationsmöglichkeiten der verschiedenen Medienkomponenten betrifft.

Durch die Aufheizung des Medienstromes 9 im Wärmetauscher 10 auf 70 °C werden einerseits viele Keime abgetötet und andererseits das Zellwachstum thermoresistenter Keime so stark vermindert, daß es nicht zu einer Überlastung von Zentrifuge 16 und Querstromfiltrationsmodul 11 kommen kann. Durch die moderate Temperatur wird eine Schädigung von Medienbestandteilen vermieden.

Der so behandelte Medienstrom 9 wird nachfolgend durch die Filtrationsmembrane 12 im Querstromfiltrationsmodul 11 in steriles Permeat 13 und keimhaltiges Retentat 14 aufgeteilt, wobei der Volumenstrom des Retentates 14 so groß gewählt werden muß, daß sich die Filtrationsmembrane 12 nicht zusetzt.

In der Zentrifuge 16 werden ständig Mikroorganismen aus dem Retentat 14 abgeschieden, bevor es als gereinigtes Retentat 17 wieder mit dem Medienstrom 9 vor Eintritt in den Wärmetauscher 10 vermischt wird. Dadurch wird die Gefahr des Zu- bzw. Durchwachsens der Filtrationsmembran erheblich reduziert. Außerdem sorgt die Zentrifuge 16 selbsttätig für die Aufrechterhaltung einer ausreichenden Rezirkulationsströmung, durch die eine unvorteilhafte Ablagerung von Mikroorganismen auf der Filtrationsmembran 12 unterbleibt. Des weiteren wird durch die Zentrifuge 16 ein ausreichender Transmembrandruck erzeugt.

Als günstigster Porendurchmesser der Filtrationsmembran wurden 0,14 Mikrometer ermittelt. Bei diesem Wert wird eine ausreichende Durchsatzleistung der Membran erreicht, und die Sterileigenschaften der Membran sind zufriedenstellend.

Die Durchsatzleistung der Zentrifuge 16 wird konstant gehalten durch die Aufrechterhaltung des Ablaufdruckes der Zentrifuge mittels eines Druckreglers 18, der die Pumpe 5, mit der die Medienkomponente 1 gefördert wird, entsprechend regelt.

Die Medienkomponente 1 hat einen überwiegenden Anteil an der Zusammensetzung des Medienstromes 9, und die Volumenströme der übrigen Medienkomponenten 2, 3, 4 werden dem geänderten Volumenstrom der Medienkomponente 1 durch Veränderung der Förderleistung der Pumpen 6, 7 und 8 mittels einer nicht dargestellten Regeleinrichtung angepaßt.

Als Zentrifuge 16 wird vorteilhafterweise ein Tellerseparator verwendet, der mit einer Einrichtung zur Ableitung der gereinigten Phase unter Druck versehen ist. Die abgeschiedenen Keime lagern sich in der Zentrifuge ab und müssen entweder manuell entfernt werden, oder sie werden bei Verwendung einer entsprechend ausgerüsteten Zentrifuge automatisch aus dieser abgelassen.

## Patentansprüche

1. Verfahren zur kontinuierlichen Zubereitung eines sterilen Nährmediums, bei dem verschiedene Medienkomponenten zu einem Medienstrom vermischt werden, der zur Beschickung eines Bioreaktors geeignet ist, **dadurch gekennzeichnet**, daß die Medienkomponenten vor der Sterilisation miteinander vermischt werden, der so erzeugte Medienstrom einem Querstromfiltrationsmodul zugeführt wird, das mit einer Filtrationsmembran versehen ist, durch die der Medienstrom aufgeteilt wird in Permeat und Retentat, das Retentat einer Zentrifuge zwecks Abscheidung von Keimen zugeführt wird und das zentrifugierte Retentat zurückgegeben wird in den Medienstrom zum Querstromfiltrationsmodul, während das Permeat dem Bioreaktor eingespeist wird.

2. Verfahren nach Anspruch 1, dadurch gekennzeichnet, daß die Temperatur des Medienstromes nach der Zugabe des Retentates durch einen Wärmetauscher auf eine Temperatur von mindestens 70 °C eingestellt wird.

3. Verfahren nach Anspruch 1 oder 2, dadurch gekennzeichnet, daß der Porendurchmesser der Filtrationsmembran maximal 0,2 Mikrometer beträgt.

4. Verfahren nach Anspruch 1 oder 2, dadurch gekennzeichnet, daß der Porendurchmesser der Filtrationsmembran 0,14 Mikrometer beträgt.

5. Verfahren nach einem der Ansprüche 1 bis 4, dadurch gekennzeichnet, daß der Volumenstrom des Retentates mindestens genau so groß ist wie der Volumenstrom des Permeates.

6. Verfahren nach einem der Ansprüche 1 bis 4, dadurch gekennzeichnet, daß der Volumenstrom des Retentates mindestens viermal so groß ist wie der Volumenstrom des Permeates.

7. Verfahren nach einem der Ansprüche 1 bis 5, dadurch gekennzeichnet, daß die Dosierung der Medienkomponenten mittels Verdrängerpumpen erfolgt.

8. Verfahren nach einem der Ansprüche 1 bis 7, dadurch gekennzeichnet, daß der Ablaufdruck der Zentrifuge konstant gehalten wird durch die Regelung des Volumenstromes einer Medienkomponente.

9. Verfahren nach Anspruch 8, dadurch gekennzeichnet, daß die Volumenströme der übrigen Medienkomponenten dem geänderten Volumenstrom der geregelten Medienkomponente angepaßt werden.

## Claims

1. Process for the continuous preparation of a sterile nutrient medium, in which different medium components are mixed to form a medium stream which is suitable for feeding to a bioreactor,**characterised in that** the medium components are mixed together prior to sterilization, the medium stream thus formed is fed to a cross-flow filtration module which is provided with a filtration membrane through which the medium stream is split up into permeate and retentate, whereby the retentate is sent to a centrifuge for removal of the bacteria and the centrifuged retentate is recycled back into the medium stream to the cross-flow filtration module and the permeate is fed to the bioreactor.

2. Process according to claim 1, characterised in that the temperature of the medium stream is adjusted to a temperature of at least 70 °C by means of a heat exchanger after the retentate has been added.

3. Process according to claim 1 or 2, characterised in that the pore diameter of the filtration membrane does not exceed 0.2 micrometers.

4. Process according to claim 1 or 2, characterised in that the pore diameter of the filtration membrane is 0.14 micrometers.

5. Process according to one of the claims 1 to 4, characterised in that the flow volume of the retentate is at least equal to the flow volume of the permeate.

6. Process according to one of the claims 1 to 4, characterised in that the flow volume of the retentate is four times greater than the flow volume of the permeate.

7. Process according to one of the claims 1 to 5, characterised in that the dosing of the medium components is by means of positive displacement pumps.

8. Process according to one of the claims 1 to 7, characterised in that the discharge pressure of the centrifuge is kept constant by regulating the flow volume of one component.

9. Process according to claim 8, characterised in that the flow volumes of the other medium components are adjusted to the changed flow volume of the regulated medium components.

## Revendications

1. Procédé de préparation d'un milieu nutritif stérile par mélange de différents composants dans le but d'obtenir un courant de milieu pouvant être alimenté à un bio-réacteur, **caractérisé en ce que** les composants du milieu sont mélangés entre eux avant stérilisation, le courant de milieu ainsi obtenu étant envoyé vers un module de filtration à courant transversal équipé d'une membrane filtrante séparant le courant de milieu dans un perméat et dans un rétentat, le rétentat étant ensuite alimenté à une centrifugeuse dans laquelle il est débarrassé des germes avant d'être recyclé dans le courant de milieu s'écoulant vers le module de filtra- rion à courant transversal, tandis que le perméat est envoyé vers le bio-réacteur.

2. Procédé selon revendication 1, caractérisé en ce que la température du courant de milieu après addition du rétentat centrifugé est réglée à au moins 70 °C par intervention d'un échangeur thermique.

3. Procédé selon les revendications 1 ou 2, caractérisé en ce que le diamètre des pores de la membrane filtrante est de 0,2 micromètre au maximum.

4. Procécé selon les revendications 1 ou 2, caractérisé en ce que le diamètre des pores de la membrane filtrante est de 0,14 micromètre.

5. Procédé selon l'une des revendications 1 à 4, caractérisé en ce que le volume du courant de rétentat est d'au moins aussi important que le volume du courant de perméat.

6. Procédé selon l'une des revendications 1 à 4, caractérisé en ce que le volume du courant de rétentat est d'au moins quatre fois la valeur du volume du couant de perméat.

7. Procédé selon l'une des revendications 1 à 5, caractérisé en ce que le dosage des composants du milieu s'effectue grâce à des pompes de déplacement.

8. Procécé selon l'une des revendications 1 à 7, caractérisée en ce que la pression de refoulement en sortie de la centrifugeuse est maintenueconstante par réglage du volume de courant de l'un des composants de milieu.

9. Procédé selon revendication 8, caractérisé en ce que les volumes des courants des autres composants de milieu sont adaptés au volume modifié du courant du composant de milieu ayant fait l'objet du réglage.
